# EUROPEAN PATENT APPLICATION

(11) **EP 4 603 843 A1**
(43) Date of publication of application: **20.08.2025**
(21) Application number: 23877386.5
(22) Date of filing: 16.10.2023
(51) Int. Cl.: G01N 35/04, G01N 21/17

(54) **INFORMATION PROCESSING DEVICE, INFORMATION PROCESSING METHOD, AND INFORMATION PROCESSING PROGRAM**

(30) Priority: 14.10.2022 JP 2022165729
(71) Applicant: ARKRAY, Inc., Kyoto-shi, Kyoto 601-8045 (JP)
(72) Inventor: NAKAO, Atsushi, Kyoto-Shi Kyoto 602-0008 (JP); FUJIMOTO, Koji, Kyoto-Shi Kyoto 602-0008 (JP); NAKAJIMA, Shinya, Kyoto-Shi Kyoto 602-0008 (JP)
(74) Representative: MFG Patentanwälte Meyer-Wildhagen Meggle-Freund Gerhard PartG mbB
(86) International application number: PCT/JP2023/037455
(87) International publication number: WO 2024/080385

(57) **Abstract**

An information processing apparatus includes an acquisition unit configured to acquire a captured image of a specimen captured by a camera provided in a portable information terminal; and an output unit configured to output the acquired captured image to a processing apparatus configured to perform processing related to measurement processing of measuring a tangible component included in the specimen based on the captured image.

## Description

### BACKGROUND

### Technical Field

The present disclosure relates to an information processing apparatus, an information processing method, and an information processing program.

### Related Art

Japanese National-Phase Publication (JP-A) No. 2016-522880 discloses a microscopic method for classifying particles as two-dimensional objects in a field of view, including steps of: a) illuminating a field of view with a first source of electromagnetic radiation; b) projecting an image obtained by an image sensor to obtain a first digital image of the field of view; c) identifying a first object in the first digital image using the first digital image; d) defining a region of the first object included in the first digital image using contour coordinates of a contour of the first object and determining a boundary line of the region of the first object; e) determining one or more object properties of the first object; f) separating, with respect to the first digital image of the first object, particles in contact by subtracting one or more pixels from a boundary; g) using the contour coordinates to define a region adjacent to the contour of the first object but outside the first object, calculating a background illumination intensity of the first source of electromagnetic radiation, and subtracting this average background illumination intensity from an electromagnetic illumination intensity of the first object; and h) using the electromagnetic illumination intensity of a region within the contour of the first object to determine a characteristic of the particles in the first object that absorb light by referencing a standard curve.

### SUMMARY OF INVENTION

Conventionally, an apparatus for measuring a tangible component of a specimen such as urine is usually provided with a camera designed exclusively for the apparatus, and there has been a problem that the cost of the apparatus increases.

The disclosure has been made in view of the above points, and an object thereof is to provide an information processing apparatus, an information processing method, and an information processing program capable of measuring a tangible component included in a specimen at a low cost.

In order to achieve the above object, an information processing apparatus according to an aspect of the disclosure includes: an acquisition unit configured to acquire a captured image of a specimen captured by a camera provided in a portable information terminal; and an output unit configured to output the acquired captured image to a processing apparatus configured to perform processing related to measurement processing of measuring a tangible component included in the specimen based on the captured image.

According to the disclosure, it is possible to obtain an effect that a tangible component included in a specimen can be measured at a low cost.

### BRIEF DESCRIPTION OF THE DRAWINGS

Fig. 1 is a configuration diagram of a measurement system according to a first embodiment;
Fig. 2 is a configuration diagram illustrating a hardware configuration of a portable information terminal according to the first embodiment;
Fig. 3 is a configuration diagram illustrating a functional configuration of the portable information terminal according to the first embodiment;
Fig. 4 is a flowchart of information processing executed by the portable information terminal according to the first embodiment;
Fig. 5 is a configuration diagram of a measurement system according to a second embodiment;
Fig. 6 is a configuration diagram illustrating a hardware configuration of a server according to the second embodiment;
Fig. 7 is a flowchart of measurement processing executed by a portable information terminal according to the second embodiment;
Fig. 8 is a flowchart of measurement processing executed by a server according to the second embodiment; and
Fig. 9 is a configuration diagram of a measurement system according to a third embodiment.
Fig. 10 is a configuration diagram of a measurement system according to a fourth embodiment.
Fig. 11A is a top view of an inspection apparatus according to the fourth embodiment.
Fig. 11B is a cross-sectional diagram taken along line A-A of Fig. 11A.
Fig. 12 is a flowchart of measurement processing executed by a portable information terminal according to the fourth embodiment.

### DETAILED DESCRIPTION OF THE INVENTION

Hereinafter, an example of a mode for carrying out the technique of the disclosure will be described in detail with reference to the drawings. Components and processing having the same operation, action, and function are denoted by the same reference numerals throughout the drawings, and redundant description may be omitted, as appropriate. Each drawing is only schematically illustrated to the extent that the technique of the disclosure can be sufficiently understood. Therefore, the technique of the disclosure is not limited only to the illustrated example. In the present embodiment, description of configurations that are not directly related to the disclosure or well-known configurations may be omitted.

### <First Embodiment>

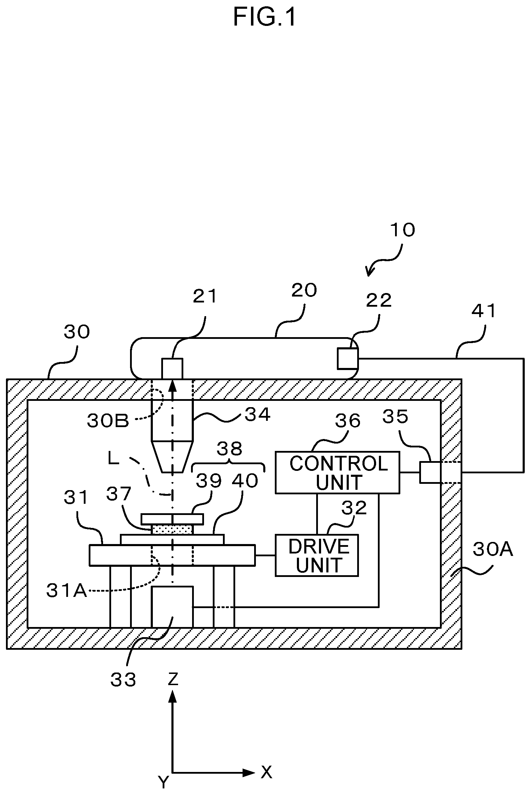

Fig. 1 illustrates a configuration of a measurement system 10 according to the present embodiment.

As illustrated in Fig. 1, the measurement system 10 includes a portable information terminal 20 and an inspection apparatus 30.

The portable information terminal 20 is a camera-equipped portable information terminal such as a smartphone including a camera 21, a communication unit 22, or the like, for example.

The inspection apparatus 30 includes a stage 31, a drive unit 32, a light source 33, an optical system 34, a communication unit 35, and a control unit 36.

A preparation 38 on which a specimen 37 to be measured is set is placed on the stage 31. The preparation 38 includes a cover glass 39 and a slide glass 40. The specimen 37 is set on the slide glass 40 and covered with the cover glass 39 from above. In the present embodiment, a case where the preparation 38 is used as a chamber for setting the specimen 37 will be described, but other chambers such as a flow cell may be used without being limited to the preparation 38.

The light source 33 is provided on a bottom surface of the inspection apparatus 30 in a housing 30A. The light source 33 emits light in a Z-axis direction in Fig. 1. The light source 33 is controlled by the control unit 36.

A passing hole 31A through which light L emitted from the light source 33 passes is provided at a center of the stage 31. The preparation 38 in which the specimen 37 is set is set at a position of the passing hole 31A. The light L emitted from the light source 33 passes through the passing hole 31A, passes through the specimen 37 of the preparation 38, and enters the optical system 34.

The optical system 34 includes an optical component such as a lens (not illustrated).

A passing hole 30B is provided on a light emitting port side of the optical system 34 from which the light L is emitted. At the time of measurement of the specimen 37, the portable information terminal 20 is set on an upper surface of the housing 30A such that a position of the camera 21 of the portable information terminal 20 coincides with a position of the passing hole 30B.

The stage 31 is driven by the drive unit 32. The drive unit 32 drives the stage 31 in an X direction, a Y direction, and a Z direction orthogonal to each other in accordance with an instruction from the control unit 36.

The communication unit 35 is provided on a side surface of the housing 30A. At the time of measurement of the specimen 37, the communication unit 22 of the portable information terminal 20 and the communication unit 35 of the inspection apparatus 30 are coupled by a communication cable 41. The portable information terminal 20 and the inspection apparatus 30 may be wirelessly coupled.

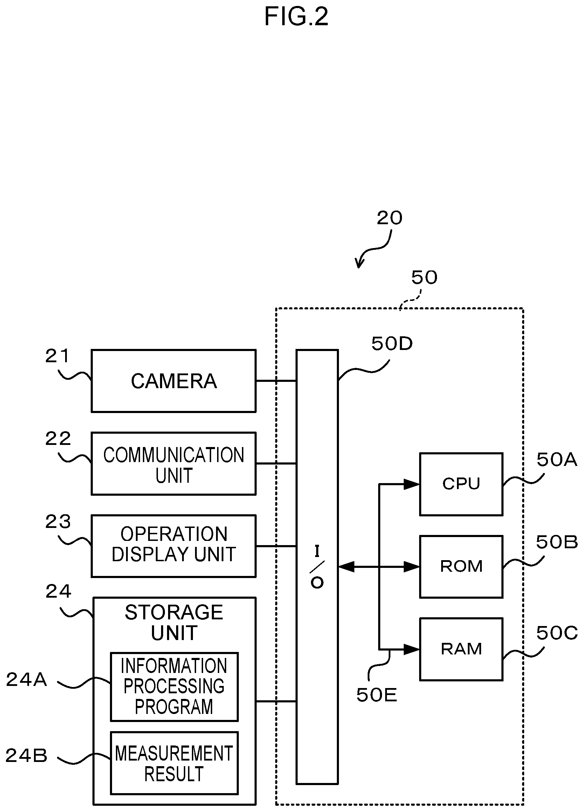

Fig. 2 is a block diagram illustrating a hardware configuration of the portable information terminal 20. As illustrated in Fig. 2, the portable information terminal 20 includes a controller 50.

The controller 50 includes a central processing unit (CPU) 50A, a read only memory (ROM) 50B, a random access memory (RAM) 50C, and an input/output interface (I/O) 50D. The CPU 50A, the ROM 50B, the RAM 50C, and the I/O 50D are coupled respectively via a bus 50E. The bus 50E includes a control bus, an address bus, and a data bus. The camera 21, the communication unit 22, the operation display unit 23, and the storage unit 24 are coupled to the I/O 50D.

The camera 21 includes an imaging element such as a charge coupled device (CCD).

The communication unit 22 is an interface for performing data communication with an external apparatus such as the inspection apparatus 30.

The operation display unit 23 includes, for example, a touch panel or the like.

The storage unit 24 includes, for example, a nonvolatile memory. As illustrated in Fig. 2, the storage unit 24 stores an information processing program 24A, a measurement result 24B of a tangible component of the specimen 37, and the like.

The CPU 50A is an example of a processor. The processor herein refers to a processor in a broad sense, and includes a general-purpose processor (for example, CPU) or a dedicated processor (for example, GPU: Graphics Processing Unit, ASIC: Application Specific Integrated Circuit, FPGA: Field Programmable Gate Array, Programmable Logic Device, and the like).

The information processing program 24A may be appropriately installed in the portable information terminal 20 by being stored in a nonvolatile non-transitory recording medium or distributed via a network.

Examples of the nonvolatile non-transitory recording medium include a compact disc read only memory (CD-ROM), a magneto-optical disk, a hard disk drive (HDD), a digital versatile disc read only memory (DVD-ROM), a flash memory, a memory card, and the like.

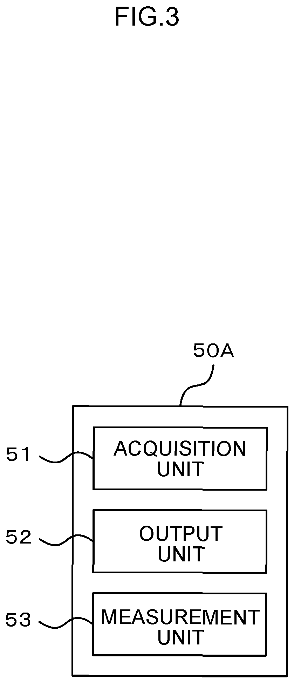

Fig. 3 is a block diagram illustrating a functional configuration of the CPU 50A of the portable information terminal 20. As illustrated in Fig. 3, the CPU 50A functionally includes functional units of an acquisition unit 51, an output unit 52, and a measurement unit 53. The CPU 50A functions as each functional unit by reading and executing the information processing program 24A stored in the storage unit 24.

The acquisition unit 51 acquires a captured image of the specimen 37 captured by the camera 21.

The output unit 52 outputs the captured image acquired by the acquisition unit 51 to the measurement unit 53 as an example of a processing unit that performs processing related to measurement processing of measuring a tangible component included in the specimen 37 based on the captured image.

The output unit 52 outputs an instruction signal for instructing the inspection apparatus 30 to drive at least one of the light source 33 and the drive unit 32.

The measurement unit 53 measures a tangible component included in the specimen 37 based on the captured image acquired by the acquisition unit 51.

In the present embodiment, a case where the specimen 37 is a urine specimen and a tangible component in the urine specimen is measured will be described. The urine specimen includes a plurality of types of tangible components. Examples of the type of the tangible component include red blood cells, white blood cells, epithelial cells, cylinders, and bacteria, for example. In the present embodiment, as an example of the specimen 37, a case of measuring an in-urine tangible component using a urine specimen will be described, but the technique of the disclosure can also be applied to a case of measuring a tangible component using blood, cells, body fluid, or the like as a specimen.

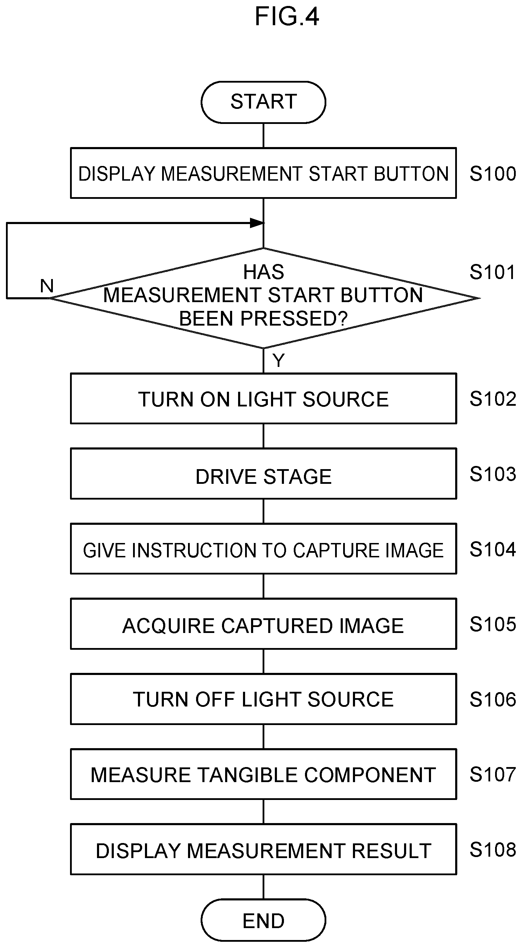

Next, an action of the portable information terminal 20 according to the present embodiment will be described with reference to Fig. 4.

Fig. 4 is a flowchart illustrating an example of a flow of processing by the information processing program 24A according to the present embodiment. The information processing illustrated in Fig. 4 is executed by a user performing an operation of instructing execution of the information processing program 24A from a menu of the portable information terminal 20. Prior to the execution of the information processing program 24A, the user places the preparation 38 on which the specimen 37 is set on the stage 31. The user sets the portable information terminal 20 on an upper surface of the housing 30A of the inspection apparatus 30 such that the position of the camera 21 coincides with the position of the passing hole 30B of the inspection apparatus 30.

In step S100, the CPU 50A displays a measurement start button on the operation display unit 23.

In step S101, the CPU 50A determines whether the measurement start button has been pressed. When the measurement start button is pressed, the process proceeds to step S102, and when the measurement start button is not pressed, the process waits until the measurement start button is pressed.

In step S102, the CPU 50A outputs an instruction signal to the inspection apparatus 30 to turn on the light source 33. As a result, the control unit 36 of the inspection apparatus 30 turns on the light source 33.

In step S103, the CPU 50A outputs an instruction signal to the inspection apparatus 30 to drive the stage 31. As a result, the control unit 36 of the inspection apparatus 30 controls the drive unit 32 to drive the stage 31 so that the preparation 38 is positioned on an optical axis of the light L.

In step S104, the CPU 50A instructs the camera 21 to capture an image. As a result, the image of the specimen 37 is captured by the camera 21.

In step S105, the CPU 50A acquires the captured image captured by the camera 21.

In step S106, the CPU 50A outputs an instruction signal to the inspection apparatus 30 to turn off the light source 33. As a result, the control unit 36 of the inspection apparatus 30 turns off the light source 33.

In step S107, the CPU 50A measures a tangible component in the specimen 37 based on the captured image acquired in step S105. Various known methods can be used to the measurement of the tangible component based on the captured image. For example, a tangible component image included in the captured image is extracted by a known image analysis method, feature amounts such as size and contrast are analyzed for each of the extracted tangible component images, and the tangible components are classified into predetermined classification items based on the analyzed feature amounts. Then, for each of the classified tangible components, the concentration and the like of the tangible component are calculated based on the number, size, and the like of the tangible component images to obtain a measurement result.

In step S108, the CPU 50A stores the measurement result of step S107 in the storage unit 24 and displays the measurement result on the operation display unit 23.

As described above, in the present embodiment, the tangible component of the specimen 37 is measured by using the camera-equipped portable information terminal 20. As a result, it is not necessary to use a camera exclusively designed for the inspection apparatus 30, and the tangible component included in the specimen 37 can be measured at a low cost.

### <Second Embodiment>

Next, a second embodiment will be described. The same portions as those of the first embodiment are denoted by the same reference numerals, and detailed description thereof will be omitted.

In the first embodiment, a case where the portable information terminal 20 executes the measurement of the tangible component of the specimen 37 has been described, but in the second embodiment, a case where a server coupled to the portable information terminal 20 via the network executes the measurement of the tangible component of the specimen 37 will be described.

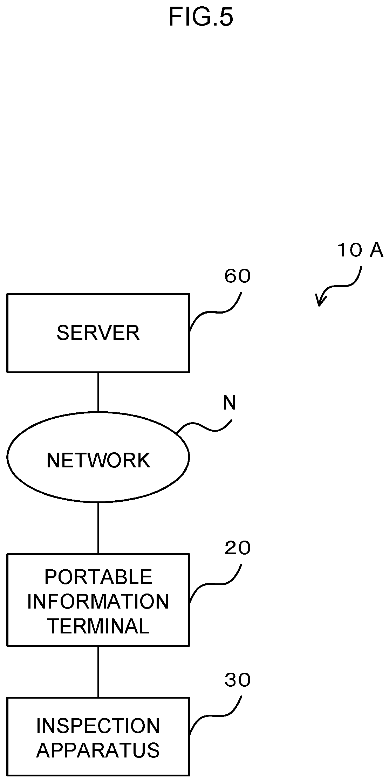

Fig. 5 illustrates a configuration of a measurement system 10A according to the second embodiment. As illustrated in Fig. 5, the measurement system 10A according to the second embodiment includes a portable information terminal 20, an inspection apparatus 30, and a server 60. The server 60 is coupled to the portable information terminal 20 via a network N. In the second embodiment, the portable information terminal 20 has the functions of the acquisition unit 51 and the output unit 52 illustrated in Fig. 3, and the server 60 has the function of the measurement unit 53 illustrated in Fig. 3. The server 60 is an example of a management apparatus of the disclosure.

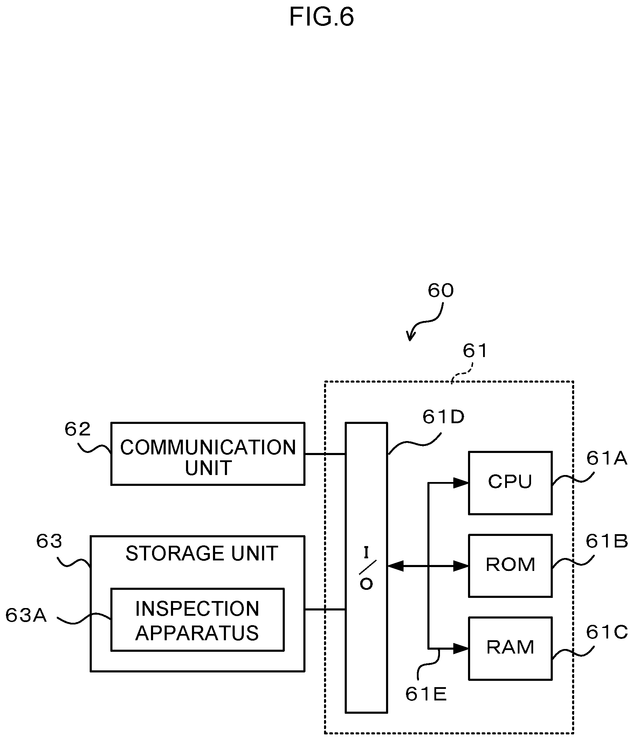

Fig. 6 is a block diagram illustrating a hardware configuration of the server 60. As illustrated in Fig. 6, the server 60 includes a controller 61.

The controller 61 includes a central processing unit (CPU) 61A, a read only memory (ROM) 61B, a random access memory (RAM) 61C, and an input/output interface (I/O) 61D. The CPU 61A, the ROM 61B, the RAM 61C, and the I/O 61D are coupled respectively via a bus 61E. The bus 61E includes a control bus, an address bus, and a data bus. A communication unit 62 and a storage unit 63 are coupled to the I/O 61D.

The communication unit 62 is an interface for performing data communication with an external apparatus such as the portable information terminal 20.

The storage unit 63 includes, for example, a nonvolatile memory. As illustrated in Fig. 6, the storage unit 63 stores a measurement program 63A.

The CPU 61A is an example of a processor as described in the first embodiment. The measurement program 63A may be appropriately installed in the server 60 by being stored in a nonvolatile non-transitory recording medium or distributed via the network, as described in the first embodiment.

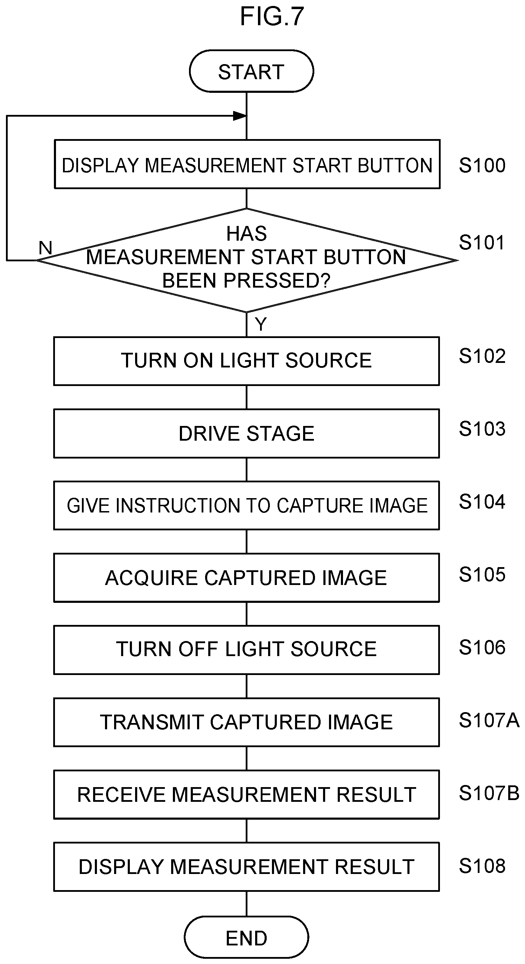

Fig. 7 is a flowchart illustrating an example of a flow of processing by the information processing program 24A according to the second embodiment executed in the portable information terminal 20. The information processing illustrated in Fig. 7 is different from the information processing illustrated in Fig. 4 in the processing of steps S107A and S107B, and the processing of the other steps is similar to the information processing illustrated in Fig. 4, and thus description thereof is omitted.

In step S107A, the CPU 50A transmits the captured image acquired in step S105 to the server 60.

In step S107B, the CPU 50A receives a measurement result of the tangible component of the specimen 37 from the server 60.

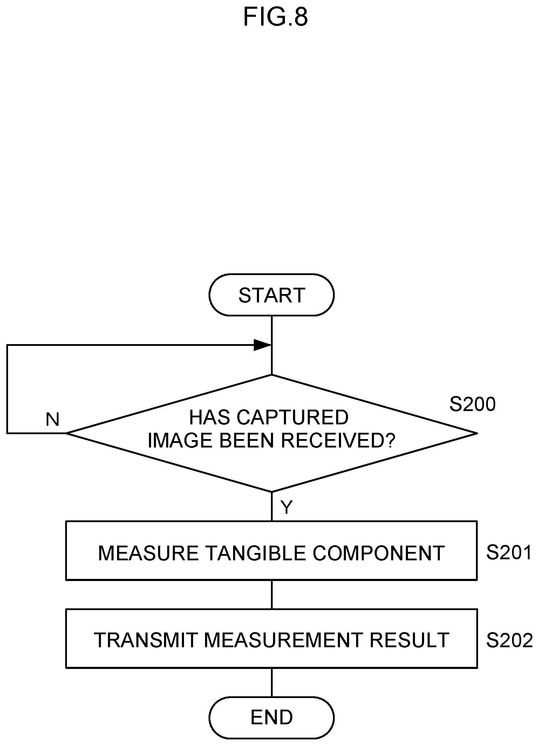

Fig. 8 is a flowchart illustrating an example of a flow of processing by the measurement program 63A executed by the CPU 61A of the server 60.

In step S200, the CPU 61A determines whether the captured image transmitted from the portable information terminal 20 has been received. When the captured image transmitted from the portable information terminal 20 is received, the process proceeds to step S201. In this regard, when the captured image transmitted from the portable information terminal 20 has not been received, the process waits until the captured image is received.

In step S201, the CPU 61A measures the tangible component of the specimen 37 based on the captured image received in step S200. This processing is similar to the processing in step S107 in Fig. 4, and thus description thereof is omitted.

In step S202, the CPU 61A transmits the measurement result of step S201 to the portable information terminal 20.

As described above, in the second embodiment, the portable information terminal 20 transmits the captured image of the specimen 37 to the server 60, and the server 60 measures the tangible component of the specimen 37. As a result, a processing load on the portable information terminal 20 can be reduced.

### <Third Embodiment>

Next, a third embodiment will be described. The same portions as those of the second embodiment are denoted by the same reference numerals, and detailed description thereof will be omitted.

In the third embodiment, a case will be described in which a captured image is provided to an analyst who analyzes the tangible component included in the specimen 37 based on the captured image of the specimen 37, and the analyst analyzes the tangible component included in the specimen 37.

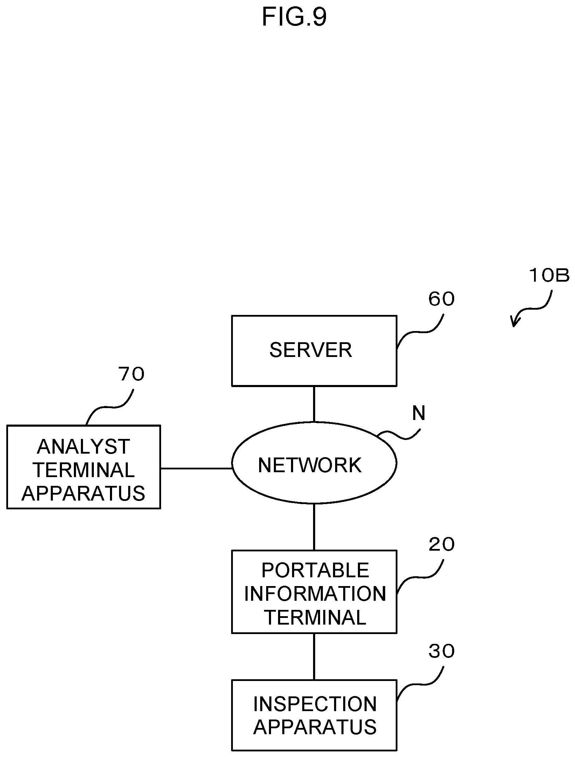

Fig. 9 illustrates a configuration of a measurement system 10B according to the third embodiment. As illustrated in Fig. 9, the measurement system 10B according to the third embodiment includes the portable information terminal 20, the inspection apparatus 30, the server 60, and an analyst terminal apparatus 70. The analyst terminal apparatus 70 is an example of an acceptance apparatus of the disclosure.

The analyst terminal apparatus 70 includes, for example, a general personal computer or the like, and has the function of the measurement unit 53 described in the first embodiment.

The portable information terminal 20 executes the information processing illustrated in Fig. 7 described in the second embodiment, but is different in that a transmission destination when the captured image is transmitted in step S107A is not the server 60 but the analyst terminal apparatus 70, and the measurement result is received from the analyst terminal apparatus 70 in step S107B.

The analyst terminal apparatus 70 executes processing similar to the processing in steps S200 and S201 in Fig. 8 described in the second embodiment. That is, when the captured image is received from the portable information terminal 20, the tangible component of the specimen 37 is measured based on the received captured image. Here, the analyst refers to the measurement result of the tangible component of the specimen 37 and performs additional analysis. Then, an additional analysis result is input to the analyst terminal apparatus 70. When the additional analysis result by the analyst is accepted, the analyst terminal apparatus 70 transmits the accepted analysis result to the portable information terminal 20. The additional analysis result may be transmitted to and stored in the server 60.

As described above, in the third embodiment, the analyst additionally analyzes the measurement result of the tangible component of the specimen 37. As a result, an accuracy of the measurement result of the tangible component of the specimen 37 can be improved.

### <Fourth Embodiment>

Next, a fourth embodiment will be described. The same portions as those in the above embodiments are denoted by the same reference numerals, and detailed description thereof will be omitted.

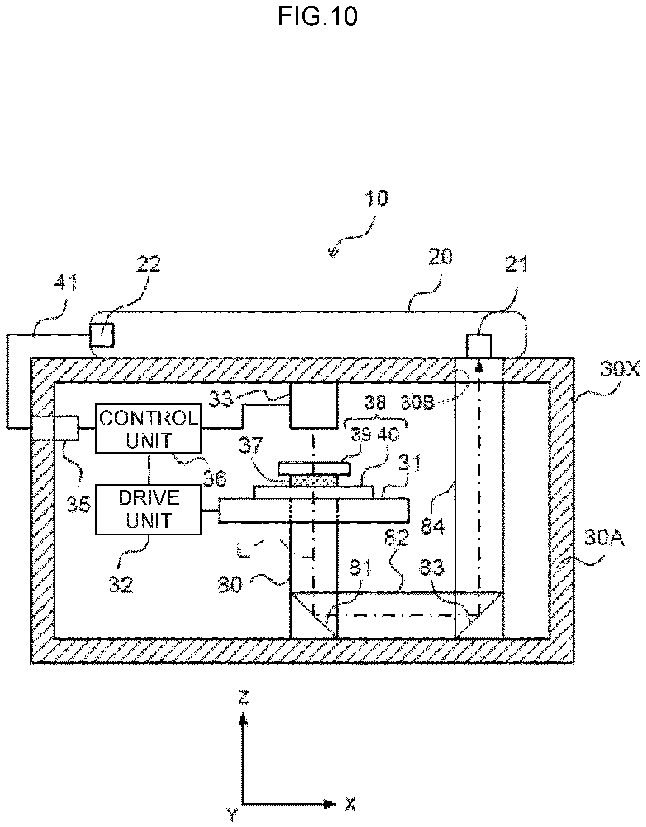

Fig. 10 illustrates an inspection apparatus 30X according to the fourth embodiment. The same portions as those of the inspection apparatus 30 in Fig. 1 are denoted by the same reference numerals, and detailed description thereof will be omitted.

The inspection apparatus 30 of Fig. 1 is configured such that the light L from the light source 33 is emitted from the lower side to the upper side of the preparation 38, and the capturing is performed from the upper side of the preparation 38.

Here, for example, in a case where the tangible component of the specimen 37 has a property of sedimentation, it may be preferable to capture an image of the specimen 37 from the lower side of the specimen 37. In addition, when focusing on a portion where a large amount of components are collected, the time until the focus is achieved may be shorter when the capturing is performed from the lower side.

Therefore, the inspection apparatus 30X of Fig. 10 according to the present embodiment is configured such that the light L from the light source 33 is emitted from the upper side to the lower side of the preparation 38, and the capturing is performed from the lower side of the preparation 38.

As illustrated in Fig. 10, the light source 33 is provided on the ceiling side of the inspection apparatus 30X. The light L emitted from the light source 33 is emitted from the upper side to the lower side along the Z axis in Fig. 1, and enters the preparation 38. The light L transmitted through the preparation 38 passes through the optical system 80 including the objective lens and is reflected rightward along the X axis in Fig. 1 by the reflecting mirror 81.

The light L reflected by the reflecting mirror 81 is reflected upward along the Z axis in Fig. 1 by the reflecting mirror 83 through the optical system 82 including an imaging lens. The light L reflected by the reflecting mirror 83 is incident on the camera 21 of the portable information terminal 20 through the optical system 84 including the eyepiece lens and the passing hole 30B.

Note that the color of the light L of the light source 33 may be either white or light bulb color. In addition, in order to avoid chromatic aberration, a light source that emits light of any one of single wavelengths of red (R), green (G), and blue (B) may be used, or a light source that emits light combining light of two wavelengths may be used.

In addition, in the preparation 38 that is usually used, the thickness of the specimen 37 that can be held is about 46 µm. However, in the operation of placing the cover glass 39 on the slide glass 40, there is a problem that there is a difference in the technique by an engineer or the number of steps is large.

Therefore, the stage 31 may be configured such that not only the preparation 38 but also other holding members such as a cuvette can be selectively placed as the holding member that holds the specimen 37, and it is possible to place two or more types of holding members that can hold the specimen 37 having different thicknesses.

A holding member that can hold a specimen 37 having a small thickness is suitable for a specimen 37 having a high component concentration in that there is no overlap or refraction of components of the specimen 37 as compared with a holding member that can hold a specimen 37 having a large thickness. In addition, it is possible to focus on the specimen 37 containing a component that do not settle easily. Conversely, the holding member that can hold a specimen 37 having a large thickness is suitable for a specimen 37 having a low component concentration because a component amount per unit area increases with sedimentation. Therefore, by adopting a configuration in which it is possible to place two or more types of holding members that can hold the specimen 37 having different thicknesses, an appropriate holding member can be used according to the component concentration of the specimen 37.

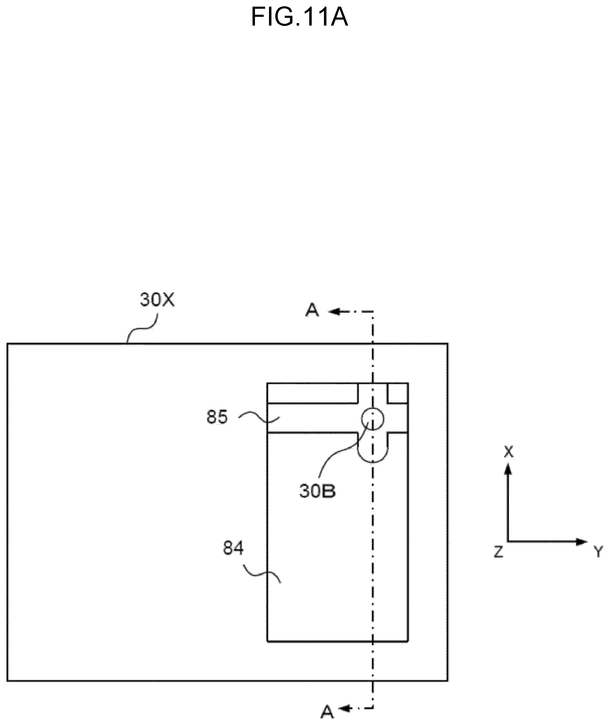

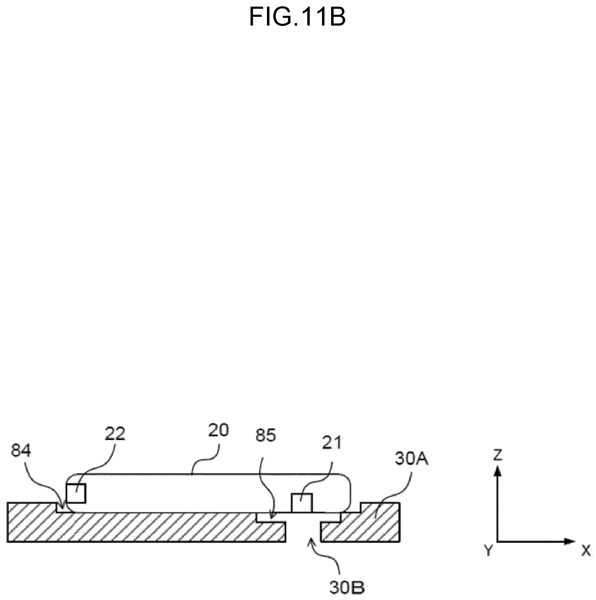

Fig. 11A is a top view of the inspection apparatus 30X. Fig. 11B is a cross-sectional diagram taken along line A-A of Fig. 11A. As illustrated in Figs. 11A and 11B, a placement portion 84 recessed in a rectangular shape according to the shape of the portable information terminal 20 is provided on the upper surface of the inspection apparatus 30X. In addition, the passing hole 30B is provided at a position of the camera 21 when the portable information terminal 20 is placed on the placement portion 84. In addition, a groove portion 85 is provided in a region along the X-axis direction and the Y-axis direction passing through the passing light 30B. Although there may be a protruding portion depending on the type of the portable information terminal 20, the presence of the groove portion 85 can prevent the portable information terminal 20 from being inclined even when there is a protruding portion in the portable information terminal 20.

When the portable information terminal 20 and the inspection apparatus 30 are wirelessly coupled by Bluetooth (registered trademark) or the like, at least a part of the placement portion 84 is preferably a member that is not metal in order to avoid occurrence of a failure in wireless communication. For example, it is preferable that the placement portion 84 is made of resin that allows radio waves to easily pass therethrough, or has a structure in which holes are appropriately formed. The passing hole 30B may be closed with a transmissive member to prevent dust and the like. In that case, the material and thickness of the transmissive member are selected in consideration of the refractive index of light and the like.

As illustrated in Fig. 11B, the placement portion 84 has such a shape that the portable information terminal 20 is placed in parallel with the upper surface of the inspection apparatus 30, and the light L enters the portable information terminal 20 at a right angle. However, there may be a case where the capturing can be favorably performed when the light is incident on the portable information terminal 20 at an angle slightly inclined from a right angle. In such a case, the shape of the placement portion 30 may be a shape in which the portable information terminal 20 is inclined with respect to the upper surface of the inspection apparatus 30.

Furthermore, the camera 21 of the portable information terminal 20 according to the present embodiment has an autofocus function. Although the autofocus function is usually turned on, it may be difficult to focus on the specimen 37.

Therefore, in the present embodiment, the output unit 52 turns off the autofocus function of the camera 21 before capturing is performed by the camera 21, and then outputs, to the drive unit 32, an instruction signal for driving the stage 31 so that the specimen 37 is in focus.

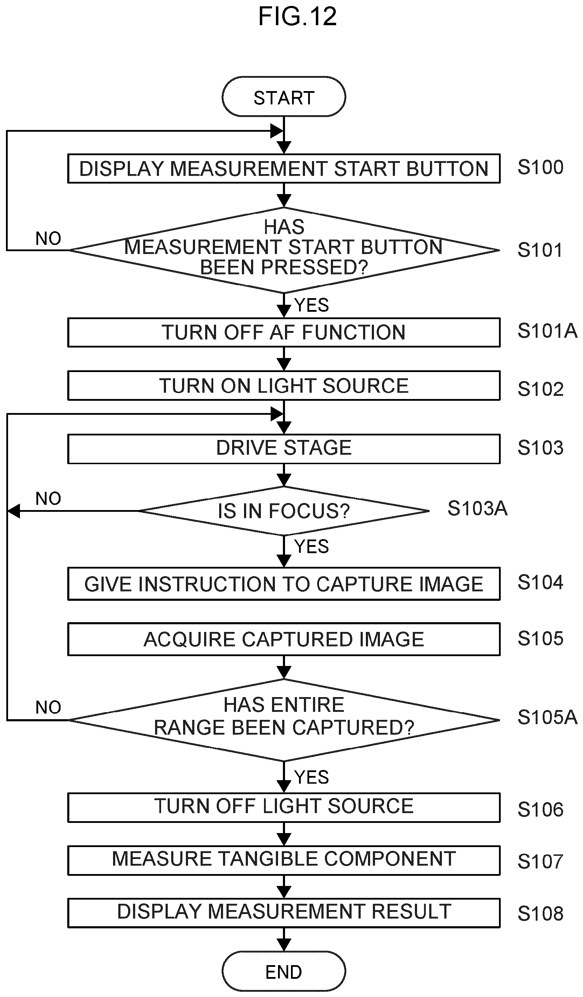

Next, an action of the portable information terminal 20 according to the present embodiment will be described with reference to Fig. 12.

Fig. 12 is a flowchart illustrating an example of a flow of information processing by the information processing program 24A according to the present embodiment. Note that steps of performing the same processing as the information processing illustrated in Fig. 4 are denoted by the same reference numerals, and detailed description thereof will be omitted.

The information processing illustrated in Fig. 12 is different from the information processing illustrated in Fig. 4 in that steps S101A, S103A, and S105A are added.

In step S101A, the CPU 50A turns off a focal position adjustment function of the camera 21. As a result, the focal position of the camera 21 is fixed at a predetermined position.

In step S103A, the CPU 50A determines whether the captured image from the camera 21 is in focus, that is, whether the specimen 37 is in focus. The determination of the focus is performed by using a known method such as a phase difference method or a contrast method.

Then, when the specimen 37 is in focus, the process proceeds to step S104. On the other hand, when the specimen 37 is out of focus, the process proceeds to step S103. In this case, in step S103, the CPU 50A detects focus information and sends the focus information to the control unit 36 of the inspection apparatus 10. As a result, the control unit 36 sends a drive instruction signal to the drive unit 32 to move the stage 31 by a predetermined amount in the Z-axis direction. In this manner, the processing of steps S103 and S103A is repeated until the specimen 37 is in focus. That is, autofocus is performed by moving and adjusting the stage 31 in the Z-axis direction instead of adjusting the focus in the camera 21.

In step S105A, the CPU 50A determines whether the entire range of the specimen 37 has been captured. Then, when the entire range of the specimen 37 has been captured, the process proceeds to step S106. On the other hand, when the entire range of the specimen 37 has not been captured, the process proceeds to step S103. In this case, in step S103, the CPU 50A drives the drive unit 31 so that the stage 31 moves in at least one of the X-axis direction and the Y-axis direction in order to move the specimen 37 to a range not yet captured. As described above, the CPU 50Arepeats the processing of steps S103 to S105A until the capturing of the entire range of the specimen 37 is completed.

As described above, in the present embodiment, the autofocus function in the focal position adjustment function of the camera 21 is turned off, and the stage 31 is driven in the Z-axis direction to perform focusing. As a result, even when it is difficult to focus on the specimen 37 by the focal position adjustment function of the camera 21, it is possible to focus on the specimen 37 by autofocus by stage driving.

In addition, the processing of step S101A may be omitted and the focal position adjustment function of the camera 21 may not be turned off, and first, in step S103A, it may be determined whether focusing is achieved by the focal position adjustment function of the camera 21. When it is determined that focusing is not achieved, the focal position adjustment function of the camera 21 may be turned off, and autofocus by the stage driving of driving the stage 31 in the Z-axis direction to perform focusing may be performed.

The operation of the processor in the above embodiment may be performed not only by one processor but also by cooperation of a plurality of processors existing at physically separated positions. An order of each operation of the processor is not limited to the order described in the above embodiment, and may be changed, as appropriate.

The configuration of the information processing apparatus described in the above embodiment is an example, and may be changed according to a situation within a scope not departing from the gist.

The flow of processing of the program described in the above embodiment is also an example, and unnecessary steps may be deleted, new steps may be added, or the processing order may be changed within the scope not departing from the gist.

In the above-described embodiment, a case where the processing according to the embodiment is realized by a software configuration using a computer by executing a program has been described, but the invention is not limited thereto. The embodiment may be realized by, for example, a hardware configuration or a combination of a hardware configuration and a software configuration.

Regarding the above embodiment, the following is further disclosed.

An information processing apparatus according to a first aspect includes an acquisition unit configured to acquire a captured image of a specimen captured by a camera provided in a portable information terminal; and an output unit configured to output the acquired captured image to a processing unit configured to perform processing related to measurement processing of measuring a tangible component included in the specimen based on the captured image.

In an information processing apparatus according to a second aspect, in the information processing apparatus according to the first aspect, the processing unit is a measurement unit that measures the tangible component included in the specimen based on the captured image, and the acquisition unit, the output unit, and the measurement unit are provided in the portable information terminal.

In an information processing apparatus according to a third aspect, in the information processing apparatus according to the first aspect, the processing unit is a measurement unit that measures the tangible component included in the specimen based on the captured image, the acquisition unit and the output unit are provided in the portable information terminal, and the measurement unit is provided in a management apparatus capable of communicating with the portable information terminal.

In an information processing apparatus according to a fourth aspect, in the information processing apparatus according to the first aspect, the processing unit is an acceptance apparatus that provides the captured image to an analyst who analyzes the tangible component included in the specimen based on the captured image and accepts an analysis result by the analyst.

In an information processing apparatus according to a fifth aspect, in the information processing apparatus according to any one of the first to fourth aspects, the output unit outputs an instruction signal giving an instruction for driving of at least one of a light source and a drive unit to an inspection apparatus including the light source that irradiates the specimen with light and the drive unit that drives a stage on which the specimen is placed.

In an information processing apparatus according to a sixth aspect, in the information processing apparatus according to the fifth aspect, the output unit turns off a focal position adjustment function of the camera before capturing is performed by the camera, and then outputs, to the drive unit, an instruction signal for driving the stage so that the specimen is in focus.

In an information processing apparatus according to a seventh aspect, in the information processing apparatus according to any one of the first to fifth aspects, the specimen is a urine specimen.

An information processing method according to an eight aspect for causing a computer to execute a process, the process including acquiring a captured image of a specimen captured by a camera provided in a portable information terminal; and outputting the acquired captured image to a processing apparatus configured to perform processing related to measurement processing of measuring a tangible component included in the specimen based on the captured image.

An information processing program according to a ninth aspect for causing a computer to execute a process, the process including acquiring a captured image of a specimen captured by a camera provided in a portable information terminal; and outputting the acquired captured image to a processing apparatus configured to perform processing related to measurement processing of measuring a tangible component included in the specimen based on the captured image.

The disclosure of Japanese Patent Application No. 2022-165729 is incorporated herein by reference in their entirety. All documents, patent applications, and technical standards described in this specification are incorporated herein by reference to the same extent as if each document, patent application, and technical standard were specifically and individually indicated to be incorporated by reference.

## Claims

1. An information processing apparatus, comprising:
an acquisition unit configured to acquire a captured image of a specimen captured by a camera provided at a portable information terminal; and
an output unit configured to output the acquired captured image to a processing unit configured to perform processing related to measurement processing for measuring a tangible component included in the specimen based on the captured image.

2. The information processing apparatus according to claim 1, wherein the processing unit is a measurement unit that measures the tangible component included in the specimen based on the captured image, and the acquisition unit, the output unit, and the measurement unit are provided at the portable information terminal.

3. The information processing apparatus according to claim 1, wherein the processing unit is a measurement unit that measures the tangible component included in the specimen based on the captured image, the acquisition unit and the output unit are provided at the portable information terminal, and the measurement unit is provided at a management device configured communicably with the portable information terminal.

4. The information processing apparatus according to claim 1, wherein the processing unit is a reception device that provides the captured image to an analyst who analyzes the tangible component included in the specimen based on the captured image and receives a result of analysis by the analyst.

5. The information processing apparatus according to claim 1, wherein the output unit outputs, to an inspection device including a light source that irradiates the specimen with light and a drive unit that drives a stage on which the specimen is placed, an instruction signal giving an instruction for driving of at least one of the light source or the drive unit.

6. The information processing apparatus according to claim 5, wherein the output unit turns off a focal position adjustment function of the camera before capture is performed by the camera, and then outputs, to the drive unit, an instruction signal for driving the stage so that the specimen is put into focus.

7. The information processing apparatus according to claim 1, wherein the specimen is a urine specimen.

8. An information processing method for causing a computer to execute processing, the processing including:
acquiring a captured image of a specimen captured by a camera provided at a portable information terminal; and
outputting the acquired captured image to a processing apparatus configured to perform processing related to measurement processing for measuring a tangible component included in the specimen based on the captured image.

9. An information processing program executable by a computer to perform processing, the processing including:
acquiring a captured image of a specimen captured by a camera provided at a portable information terminal; and
outputting the acquired captured image to a processing apparatus configured to perform processing related to measurement processing for measuring a tangible component included in the specimen based on the captured image.
